# EUROPEAN PATENT APPLICATION

(11) **EP 3 366 155 A1**
(43) Date of publication of application: **29.08.2018**
(21) Application number: 17157875.0
(22) Date of filing: 24.02.2017
(51) Int. Cl.: A44C 5/14, A44C 5/16, A44C 5/24

(54) **WATCH STRAP BUCKLE HOLDING WEARABLE MODULAR ELECTRONIC DEVICE**

(71) Applicant: Manufacture Modules Technologies Sarl, 1228 Plan-les-Ouates (CH); Frédérique Constant S.A., 1228 Plan-les-Ouates (CH)
(72) Inventor: FRABOULET, Philippe, 1228 Plan-les-Ouates (CH); KOESLAG, Pim, 1228 Plan-les-Ouates (CH); DA SILVA MATOS, Manuel, 1228 Plan-les-Ouates (CH)
(74) Representative: Weihs, Bruno Konrad

(57) **Abstract**

The invention provides a wearable smart device for a wristwatch strap or bracelet, comprising a water tight casing, and an electronic module enclosed in the water tight casing. The wearable module is distinct from an intended watch body to be mounted on the wristwatch strap or bracelet. The wearable smart device comprises on each of 2 opposite sides of the water tight casing a butterfly clip mechanism with a hinge and two brackets articulated around the hinge, whereby for each butterfly clip mechanism a first one of the two bracket is fixedly mounted on the corresponding side of the water tight casing at a part of the first bracket opposite from the hinge, and a second one of the two brackets comprises means to attach an extremity of an intended wristwatch strap or bracelet at a part of the second bracket opposition from the hinge.

## Description

### TECHNICAL FIELD

The present invention relates to a strap, a bracelet, a band, a wristwatch and wristwatch parts that contain at least one of electronics and a wireless communication system.

### BACKGROUND ART

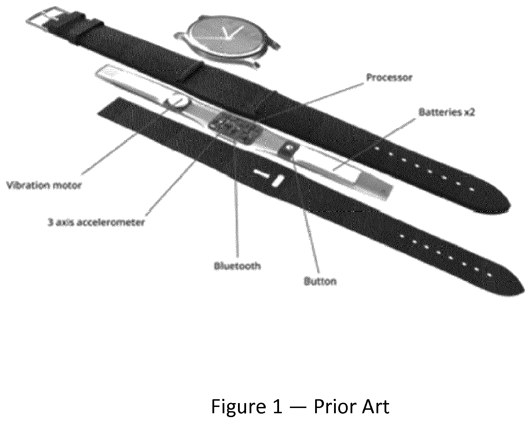

Devices with embedded sensors and wireless communication that are arranged in the watch, under the watch head or in the strap are known from prior art. One example is the watch and strap offered under the name *Classi* on web page www.maintool.me. The *Classi* product is configured to upgrade a classic watch by replacing the conventional strap by a smart strap that gives the watch smart features. Figure 1 illustrates the *Classi* strap and a wristwatch clock in an exploded vievv. The *Classi* integrates a vibration motor, a 3-axis accelerometer, a Bluetooth^{®} communication module, a processor, an actuator button and batteries into a classic leather watch strap. According to the manufacturer *Classi* enables to track the user's activity, receive notifications via vibration, navigate to a destination handsfree and send out for help with the push of the button. *Classi* also has a phone loss prevention feature and will vibrate strongly if the user is forgetting his phone. The smart strap interacts with a dedicated application executed on an associated smartphone device. The application allows to configure the different settings to run the notifications and displays all the data collected by means of the smart strap device. In addition, the application allows to activate a navigation mode and choose a destination to which the user is heading. However in the design of this product, the electronic modules, sensors and batteries are enclosed inside a classical leather strap thereby making the strap thicker than the initial strap. Since the strap also passes under the body of the watch the overal thickness of the wristwatch is increased as compared to the classical wristwatch. Furthermore the strap may be subject to extreme bending which in turn may compromise the integrity of the enclosed electronic modules, sensors and/or battery, and possibly also break any watertight sealing through undue mechanical stress.

US publication US 2016/029778 A1 discloses a further design for a wristwatch in which a dedicated housing contains electronic elements and battery, whereby the housing superposes over the strap, thereby increasing the overall thickness of the strap. This is a disadvantage since watchmakers have always tried to make watches as thin as possible.

The web site http://www.biowatch.ch/web/ discloses a further design for a wristwatch in which a dedicated housing contains electronic elements and battery, whereby the housing also superposes over the strap in a closed position.

Still another example of a dedicated housing in a wristwatch superposing the housing over the strap when closed, is disclosed on the web site https://www.wareable.com/sony/sony-seeks-crowdfunding-for-the-wena-smartwatch-1604. One particularity of the disclosed design is that it comprises a single butterfly hinge mechanism that folds under the housing when the strap is closed.

Hence one of the problems to be solved by the present invention, is to add electronics functions to a watch, including for example wireless connectivity, in a manner that works with most types of watches, further in a manner that is water resistant, preserves an horological look of the watch and resists to mechanical stress, while addressing inconveniences experienced in prior art devices, such as changes to the appearance of the watch, or increased thickness of the watch body and/or strap when components are located between the wrist and the watch head.

### SUMMARY OF THE INVENTION

In a first aspect the invention provides a wearable smart device for a wristwatch strap or bracelet, comprising a water tight casing, and an electronic module enclosed in the water tight casing. The wearable module is distinct from an intended watch body to be mounted on the wristwatch strap or bracelet. The wearable smart device comprises on each of 2 opposite sides of the water tight casing a butterfly clip mechanism with a hinge and two brackets articulated around the hinge, whereby for each butterfly clip mechanism a first one of the two bracket is fixedly mounted on the corresponding side of the water tight casing at a part of the first bracket opposite from the hinge, and a second one of the two brackets comprises means to attach an extremety of an intended wristwatch strap or bracelet at a part of the second bracket opposition from the hinge.

In a second aspect the invention provides a wristwatch comprising a wristwatch strap or bracelet; a watch body functionaly mounted to the wristwatch strap or bracelet, leaving two extremities of the wristwatch strap or bracelet available; and the wearable smart device of the first aspect of the invention. Each one of the two available extremities of the wristwatch strap or bracelet is attached at the respective means to attach the extremety of the wristwatch strap or bracelet of the second one of the two brackets from each butterfly mechanism. When both butterfly mechanisms are in a closed position intended for wearing the wristwatch on a wrist, the extremities of the wristwatch strap or bracelet become adjacent to the water tight casing on a respective one of the 2 opposite sides, and further in the closed position, a combined thickness of the wristwatch strap or bracelet and the butterfly mechanism to which it is attached is substantially the same as a thickness of the water tight casing.

In a preferred embodiment the electronic module is configured to execute a plurality of electronic functions; and the wearable smart device further comprises at least a sensor functionally connected to a processor of the wearable smart device (22) to enable the implementation of the plurality of electronic functions which comprise at least one of the following list:
- step counting using a step counting algorithm, whereby the at least one sensor comprises an accelerometer component,
- sleep monitoring using a sleep monitoring algorithm, whereby the at least one sensor comprises the accelerometer component,
- temperature measurement using one of the at least one sensors as appropriate,
- humidity measurement using one of the at least one sensors as appropriate,
- heart rate measurement using one of the at least on sensors as appropriate, and a heart rate monitoring algorithm, and
- UV measurement using one of the at least one sensors as appropriate.

The device further comprises a processor, and at least a sensor functionally connected to the processor to enable the implementation of the plutality of electronic functions.

In a further preferred embodiment, the wearable smart device further comprises a pusher button mounted on the water thight casing, which is configured to enable interaction of an intended user with the wearable smart device, whereby the wearable smart device is configured to react to different patterns of pressing of the pusher button, to trigger and launch in the wearable smart device at least an electronic function corresponding to the patterns of pressing.

In a further preferred embodiment, the electronic module further comprises a PCB which is configured such that at the pressing of the pusher button, an electronic contact is created on the PCB and this electronic contact sends an information to the processor.

In a further preferred embodiment the wearable smart device comprises a wireless communication interface which is configured to enable communication between the wearbale smart device and a intended companion mobile application running on a distinct terminal.

In a further preferred embodiment, the wearable smart device further comprises a wireless communication interface which is configured to enable communication between the wearable smart device and an intended companion application running on a distinct terminal.

In a further preferred embodiment, the wearable smart device further comprises a processor; and at least a sensor functionally connected to the processor. The processor is configured to conduct a bidirectional communication by means of the wireless communication interface, the bidirectional communication enabling an intended user of the intended companion application to configure the wearable smart device and to read data measured by means of the at least one sensor.

In a further preferred embodiment the processor is further configured to send action information to the intended companion application, that triggers the intended companion application to execute a corresponding action.

In a further preferred embodiment, the connection means comprises at least a connection pin.

In a further preferred embodiment, the wearable smart device further comprises a rechargeable battery enclosed in the water tight casing.

In a further preferred embodiment, a first one of the poles of the rechargeable battery is permanently accessible from an outside of the water tight casing. The wearable smart device further comprises a pusher button mounted on the water thight casing, which is configured as follows: when the pusher button is un-pressed, a second one of the poles of the rechargeable battery is electrically isolated from an outside of the water tight casing; when the pusher button is pressed it is in contact with the second one of the poles and thereby establishes an electrical contact between the second one of the poles and the outside of the water tight casing.

In a further preferred embodiment, a width of the watertight casing corresponds substantially to a width of the strap.

### BRIEF DESCRIPTION OF THE DRAWINGS

The invention will be better understood through the detailed description of preferred embodiments and in reference to the figures, wherein
figure 1 shows a smart strap watch according to prior art,
figure 2 shows a typical wristwatch according to prior art,
figures 3A-3H illustrate a preferred embodiment of the invention in an closed state of the wristwatch strap or bracelet-figures 3A-3D-and in an open state of the wristwatch strap or bracelet-figures 3E-3H,
figure 4 shows an exploded view of a water tight case of a wearable smart device according to a preferred embodiment of the invention,
figure 5 shows a block diagram of the electronics in the wearable smart device according to a preferred embodiment of the invention,
figure 6a shows a diagram of the water tight case of the wearable smart device, showing a case, inside electronics, a rechargeable battery and battery contacts according to a preferred embodiment of the invention,
figure 6b shows a diagram of the ware tight case of the wearable smart device of figure 6a when it is clipped in a charger,
figure 7 a preferred embodiment of the invention in which he pusher button is implemented in a buckle case, and
figure 8, shows a preferred embodiment of the invention, comprising an example implementation of a pusher button as a pusher button and a rechargeable battery contact, in a wrist watch case.

### DESCRIPTION OF PREFERRED EMBODIMENTS OF THE INVENTION

To solve at least the above discussed problem, the wireless connectivity electronics and any electronics implementing the functions are put in a hard, water tight case which may become an extended part of the wristwatch strap or bracelet. The water tight case comprises attached to 2 of its sides which are opposite sides, butterfly clip mechanisms. On each butterfly clip mechanism there is a means to attach an extremity of an intended wristwatch strap.

A special mechanism enables to give access to the battery contacts when charging and isolating them from the outside when not charging.

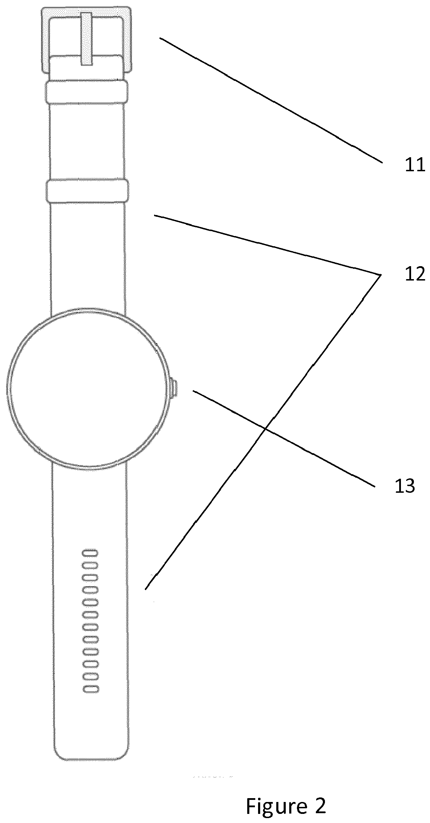

Looking at figure 2, this represents a typical watch, more particularly a wristwatch, in which a wristwatch body 13 is shown together with a strap 12 attached to the body and configured to wear the wristwatch on the wrist of an intended user (not shown in figure 2) by means of a buckle 11 located at one extremity of the strap 12.

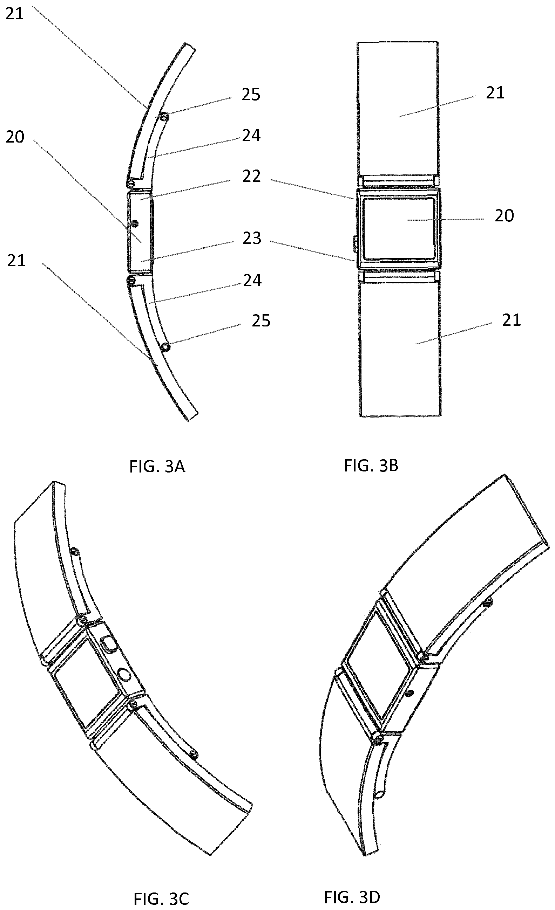

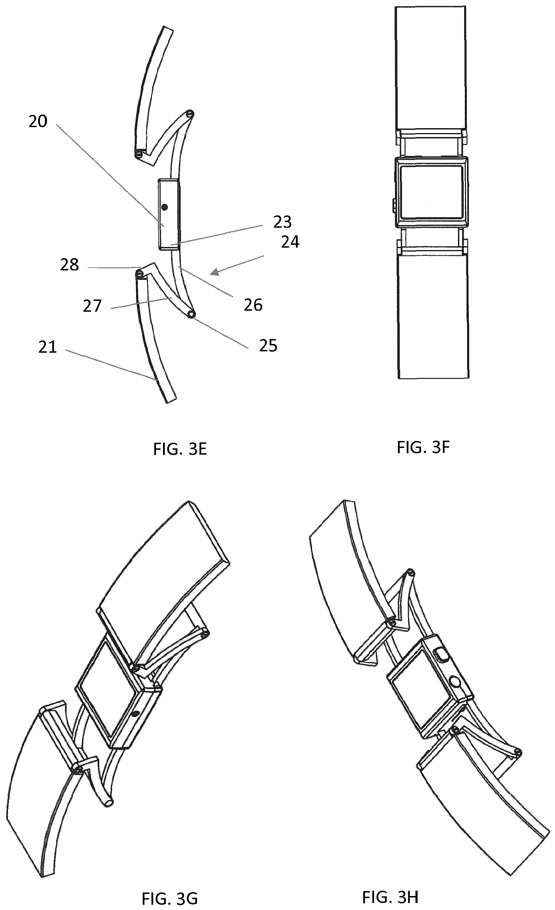

Referring to figures 3A-3H, these illustrate a preferred embodiment of the invention in an closed state of the wristwatch strap or bracelet-figures 3A-3D-and in an open state of the wristwatch strap or bracelet-figures 3E-3H.

Looking first at figure 3A, this illustrates a side view of the preferred embodiment, wherein a wearable smart device comprises a water tight casing 20 which encloses an electronic module (not shown in the figure). The wearable smart device is distinct from an intended watch body (not shown in the figure) to be mounted on the wristwatch strap or bracelet 21. It is noted that the wristwatch strap or bracelet 21 are not part of the wearable smart device and that the wearable smart device is intended to be attached to the wristwatch strap or bracelet with appropriate means explained hereinafter. The wearable smart device comprising on each of 2 opposite sides 22 and 23 of the water tight casing 22 a butterfly clip mechanism 24 with a hinge 25.

Figure 3B illustrates a view taken from above of the preferred embodiment of figure 3A. The hinges 25 are not visible as they are hidden by the wristwatch strap or bracelet 21.

Figures 3C and 3D illustrate 3-dimensional views of the preferred embodiment from figure 3A in 2 different perspectives.

Figure 3E illustrates a side view of the preferred embodiment, the same as in figure 3A, the difference being in the state of the wristwatch strap or bracelet, i.e., instead of being closed it is now open. Accordingly, further parts of the butterfly mechanism 24 may be seen. For example, figure 3E shows that each of the butterfly mechanisms comprises the hinge 25, and two brackets 26 and 27 articulated around the hinge 25. The bracket 26, here also named the first one, is fixedly mounted on the corresponding side 23 of the water tight casing 20. The part of the bracket 26 mounted to the watertight casing 20 is opposite from the hinge 25. The bracket 27, here also named the second one, comprises means to attach 28 an extremtiy of the wristwatch strap or bracelet 21. The means to attach 28 are located on the bracket 27 at a side opposite from the hinge 25.

Similarly as figures 3B-3D, the figures 3F-3H show other views of the preferred embodiment of figure 3E instead of 3A, but in different perspectives, i.e., upper view, and 3-dimensional perspectives.

In a preferred embodiment the invention relates to wristwatch, which comprises a wristwatch strap or bracelet 21, which is illustrated in its extremeties in figures 3A-3H. A watch body (not illustrated) is functionaly mounted to the wristwatch strap or bracelet 21, leaving two extremities of the wristwatch strap or bracelet available. Each one of the two available extremities of the wristwatch strap or bracelet is attached at the respective means to attach 28 (see figure 3E) of the two brackets from each butterfly mechanisms. As shown in figure 3A, when both butterfly mechanisms are in a closed position intended for wearing the wristwatch on a wrist, the extremities of the wristwatch strap or bracelet become adjacent to the water tight casing 20 on a respective one of the 2 opposite sides 22 and 23. In this closed position, a combined thickness of the wristwatch strap or bracelet 21 and the butterfly mechanism 24 to which it is attached is substantially the same as a thickness of the water tight casing 20.

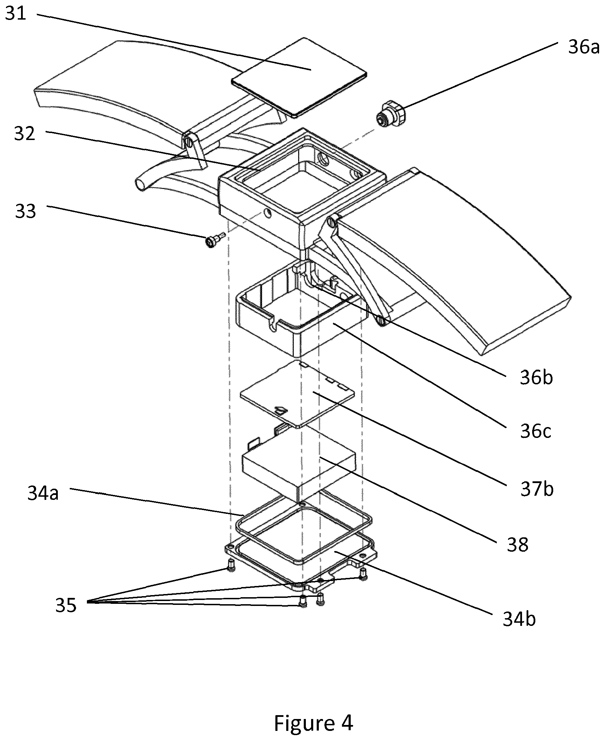

Looking at Figure 4, this shows the electronics device-or wearable smart device-according to an example of the invention, which comprises a plurality of parts, including a case comprising:
- a body 32,
- a cover 31,
- a case back 34b,
- a battery contact 33, and
- a pusher button 36a.

An electronics module, such as for example an electronics PCB 37a fits in a plastic holder 37b.

The body 32 further comprises a battery 38. In a preferred embodiment the battery 38 is of a rechargeable type.

The case is closed by means of screws 35 and water resistance is ensured by means of a case back joint 34a.

The body 32 and the case back 34b may for example be made from one or more of materials of the following list comprising metal, glass fiber, and plastic.

The cover 31 may for example be made from one or more of materials from the following list comprising glass, fiber glass, and plastic.

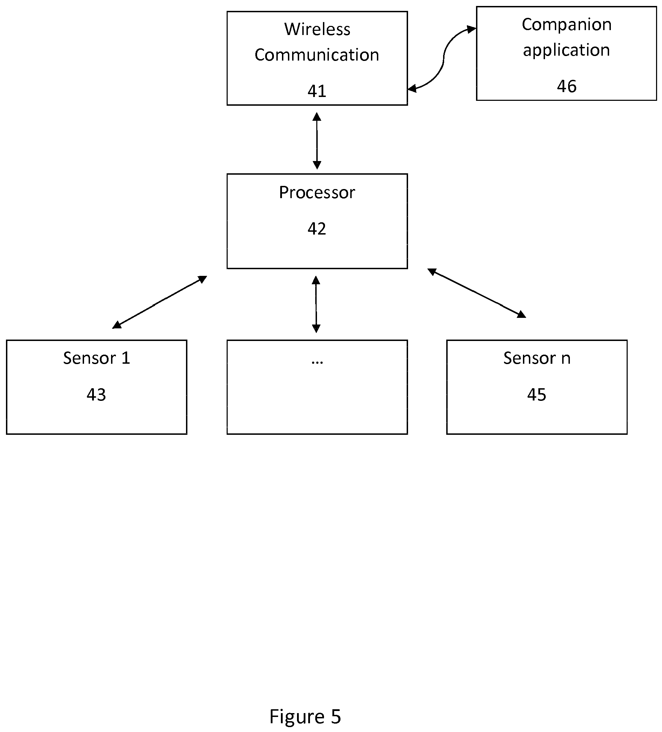

Referring to the block diagram of figure 5, this illustrates examples of electronic functions that may be realized by the PCB 37b (the PCB is not shown as such in figure 5), at least including:
- a wireless communication interface 41,
- at least one or a plurality of sensors 43-45, which may for example be biometric sensors, and
- sensor analysis and treatment implemented by an embedded processor 42.

In a preferred embodiment, the wireless communication interface 41 is a Bluetooth^{®} Low Energy type interface, that comprises a Bluetooth^{®} Low Energy chip and a ceramic antenna (not shown in the figures). The wireless communication interface 41 enables the wearable smart device to communicate with a smartphone or a tablet (not illustrated in the figures), whereby the communication on the smartphone or a tablet is conducted by a companion mobile application 46 running on the smartphone or the tablet.

Sensor measurements using sensors 43-45 may for example be:
- step counting using an accelerometer component (not shown in the figure 5) and a step counting algorithm,
- sleep monitoring using the accelerometer component and a sleep monitoring algorithm,
- temperature measurement using one of the electronic sensors 43-45 as appropriate,
- humidity measurement using one of the electronic sensors 43-45 as appropriate,
- heart rate measurement using one of the electronic sensors 43-45 as appropriate, and a heart rate monitoring algorithm, and
- UV measurement using one of the electronic sensors 43-45 as appropriate.

Via the wireless communication interface 41, the processor 42 communicates with the companion application 46 running on the smartphone or the tablet. The companion application 46 enables an interface for the user who may configure the wearable smart device and see sensor data thanks to it. The wearable smart device uses the companion application 46 as a way to communicate with the smartphone or the tablet, to get data and trigger actions.

Referring again to figure 4, when the pusher button 36a is pressed, it creates an electrical contact on the electronic PCB 37b, which triggers the sending of an information to the processor 42 of figure 5. The processor 42 interacts with the sensors 43, 45 and with the wireless communication interface 41 to launch different actions. According to the configuration of the processor 42, one of the following action may be launched:
- synchronizing sensor data with the companion application,
- changing activity recording mode, e.g., on of day or night mode,
- make the intended smartphone ring,
- trigger a camera of the smartphone,
- make the smartphone send a message or email, and
- launching an other action in the smartphone or the tablet, through the companion application 46.

In a preferred embodiment, when the pusher button 36a is pressed more than 5 seconds or when the internal accelerometer or movement sensor (not shown in figure 5) records a drop or fall, the wearable smart device transmits the information to the companion application so it can send an alarm message to the concerned people.

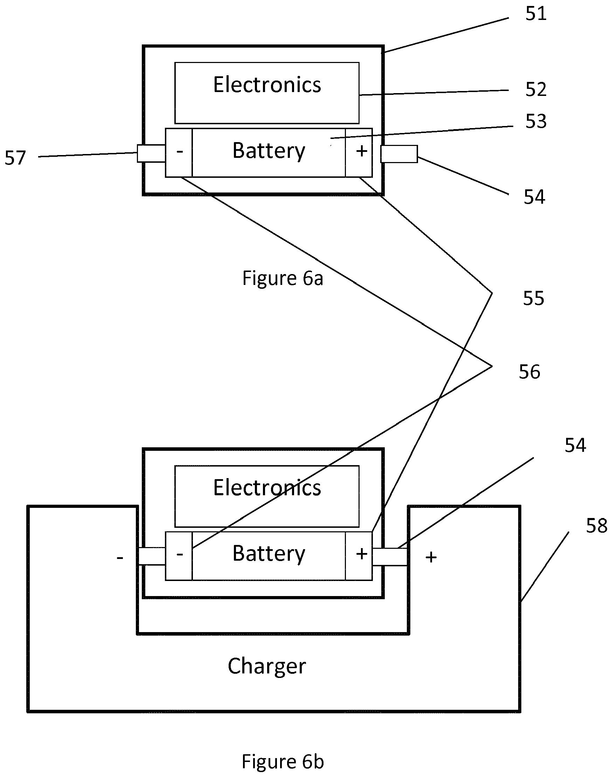

Figure 6a shows a diagram of a case 51 of the wearable smart device according to a preferred embodiment. The case is water resistant. When the pusher button 54 is not pressed-this case is represented in figure 6a-the positive contact 55 of the rechargeable battery 53 is isolated from the case 51 and from the outside. Only the negative contact 56 is accessible from the outside via a metal contact 57 (corresponding to contact 33 of figure 4). Thus, even in water, the contacts of the battery are isolated and no short-circuit is possible.

Figure 6b shows a diagram of the wearable smart device when it is plugged in a charger pod 59 as appropriate. In this position the pusher button 54-corresponding to pusher button 36a of figure 4-is pushed and a contact exists between the pusher button 54 and the positive battery contact 55. Accordingly the pusher button 54 may be made out of a conductive material, such as a metal. Thus the charger 58 has access to both contacts 55, 56 of the rechargeable battery 53 and can recharge it.

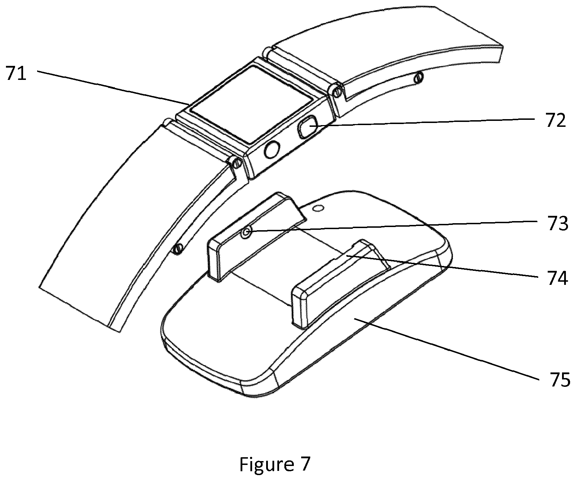

Figure 7 illustrates an implementation of the pusher button in a water tight case embodiment 71. The external contacts of the case, including the positive contact-which is not visible in figure 7-and the negative contact 33 (not visible on Figure 7, see figure 4) are on the sides of the water tight case 71. When the water tight case 71 is placed in the charger pod 75, the external contacts of the case, including negative contact 33 (not visible on Figure 7, see figure 4) are connected respectively with charger pod contacts 73 and 74. When the bwater tight case 71 is placed in the charger pod 75, the pusher button is in contact with the positive contact of the battery (not visible in figure 7, but the principle is seen in figure 6b) and thus enables the charging of it by the charger pod 75.

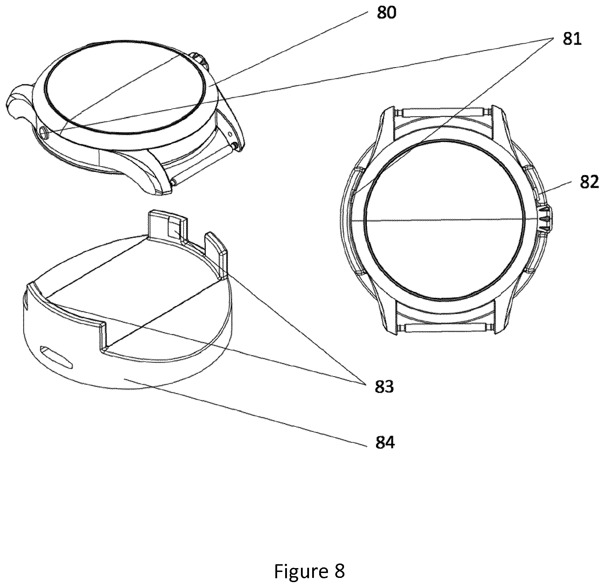

Figure 8 illustrates an other implementation of a pusher button as a pusher button and a rechargeable battery contact, in a wrist watch case 80. The watch case 80 has two contacts, and a pusher button 82. The contacts comprise a negative contact 81. The negative contact 81 is connected to the negative contact of the rechargeable battery (not shown in figure 8) inside the case 80. The pusher button 82 when it is un-pressed is not connected to the positive contact of the rechargeable battery (not visible in figure 8). When the watch is in a charger pod 84, the pusher button 82 is pressed and gets in contact with the positive contact of the internal rechargeable battery (not visible in figure 8). Thus both contacts 83 of charger pod 84 are in contact with the contacts of the case 81, including the pusher button 82 which is a contact too, and in contact with the contacts of the internal rechargeable battery (not visible in figure 8). So the charging of the battery is made possible. When the button is un-pressed, such as for example upon removal of the case 80 out of the charger pod 84, the rechargeable battery contacts are isolated from the water tight casing 80 and the system remains waterproof.

The electronics device 22 is configured to have a thickness that substantially corresponds to that of a conventional strap 21's thickness or is less than the conventional strap's thickness. In addition the electronics device 22 is configured to have a width substantially the same as that of the conventional strap. In addition the electronics device 22 may be configured to be attached to the strap and the buckle using conventional means such as the pins 23. The wearable smart device 22 is further configured to form an elongation of the wristwatch strap 21 at the time of intended wearing of the wristwatch strap by an intended user, such that at the time of intending wearing the overall thickness of the wristwatch strap and the wearable smart device remains substantially the same or less than a thickness of the wristwatch strap.

With the described invention, the position and appearance of the watch head of the wristwatch are not impacted and remain the same as in a conventional wristwatch. Moreover the electronics fit inside the water resistant hard case of the wearable smart device 22. The mechanical and humidity stress exerted on the content of the hard case remains minimal, which is better for life time of the product.

## Claims

1. A wearable smart device for a wristwatch strap or bracelet, comprising
a water tight casing;
an electronic module enclosed in the watertight casing;
the wearable module being distinct from an intended watch body to be mounted on the wristwatch strap or bracelet;
the wearable smart device comprising on each of 2 opposite sides of the water tight casing a butterfly clip mechanism with a hinge and two brackets articulated around the hinge, whereby for each butterfly clip mechanism a first one of the two bracket is fixedly mounted on the corresponding side of the water tight casing at a part of the first bracket opposite from the hinge, and a second one of the two brackets comprises means to attach an extremety of an intended wristwatch strap or bracelet at a part of the second bracket opposition from the hinge.

2. A wristwatch comprising
a wristwatch strap or bracelet;
a watch body functionaly mounted to the wristwatch strap or bracelet, leaving two extremities of the wristwatch strap or bracelet available;
the wearable smart device of claim 1;
whereby each one of the two available extremities of the wristwatch strap or bracelet is attached at the respective means to attach the extremety of the wristwatch strap or bracelet of the second one of the two brackets from each butterfly mechanism,
whereby when both butterfly mechanisms are in a closed position intended for wearing the wristwatch on a wrist, the extremities of the wristwatch strap or bracelet become adjacent to the water tight casing on a respective one of the 2 opposite sides, and further
wereby in the closed position, a combined thickness of the wristwatch strap or bracelet and the butterfly mechanism to which it is attached is substantially the same as a thickness of the water tight casing.

3. The wearable smart device (22) of claim 1, wherein
the electronic module (37b) is configured to execute a plurality of electronic functions which comprise at least one of the following list:
- step counting using a step counting algorithm, whereby the at least one sensor comprises an accelerometer component,
- sleep monitoring using a sleep monitoring algorithm, whereby the at least one sensor comprises the accelerometer component,
- temperature measurement using one of the at least one sensors (43-45) as appropriate,
- humidity measurement using one of the at least one sensors (43-45) as appropriate,
- heart rate measurement using one of the at least on sensors (43-45) as appropriate, and a heart rate monitoring algorithm, and
- UV measurement using one of the at least one sensors (43-45) as appropriate; and the wearable smart device (22) further comprises
a processor (42);
at least a sensor (43-45) functionally connected to the processor (42) to enable the implementation of the plurality of electronic functions.

4. The wearable smart device of claim 3, further comprising a pusher button (36a) mounted on the water thight casing (32), which is configured to enable interaction of an intended user with the wearable smart device (22), whereby the wearable smart device (22) is configured to react to different patterns of pressing of the pusher button (36a), to trigger and launch in the wearable smart device at least the electronic function corresponding to the patterns of pressing.

5. The wearable smart device of claim 4, whereby the electronic module further comprises a PCB (37a) which is configured such that at the pressing of the pusher button (36a), an electronic contact is created on the PCB (37a) and this electronic contact sends an information to the processor (42).

6. The wearable smart device of claim 1, further comprising a wireless communication interface (41) which is configured to enable communication between the wearable smart device and an intended companion application running on a distinct terminal.

7. The wearable smart device of claim 6, further comprising
a processor (42);
at least a sensor (43-45) functionally connected to the processor (42);
whereby the processor (42) is configured to conduct a bidirectional communication by means of the wireless communication interface (41), the bidirectional communication enabling an intended user of the intended companion application to configure the wearable smart device and to read data measured by means of the at least one sensor.

8. The wearable smart device of claim 7, whereby the processor is further configured to send action information to the intended companion application, that triggers the intended companion application to execute a corresponding action.

9. The wearable smart device of claim 1, wherein the connection means (23) comprises at least a connection pin.

10. The wearable smart device of claim 1, further comprising a rechargeable battery (38) enclosed in the watertight casing (32).

11. The wearable smart device of claim 10, wherein a first one of the poles of the rechargeable battery is permanently accessible from an outside of the watertight casing (32),
the wearable smart device (2) further comprising a pusher button (36a) mounted on the water thight casing (32), which is configured as follows:
when the pusher button (54) is un-pressed, a second one of the poles of the rechargeable battery is electrically isolated from an outside of the water tight casing (32);
when the pusher button (54) is pressed it is in contact with the second one of the poles and thereby establishes an electrical contact between the second one of the poles and the outside of the water tight casing (32).

12. The wearable smart device device of claim 1, whereby a width of the water tight casing (32) corresponds substantially to a width of the strap (21).
